# EUROPEAN PATENT APPLICATION

(11) **EP 2 251 329 A1**
(43) Date of publication of application: **17.11.2010**
(21) Application number: 09717293.6
(22) Date of filing: 03.03.2009
(51) Int. Cl.: C07D 213/73, C07D 213/82

(54) **PROCESS FOR PRODUCTION OF 3-AMINO-2-CHLORO-6- TRIFLUOROMETHYLPYRIDINE**

(30) Priority: 04.03.2008 JP 2008053247
(71) Applicant: ISHIHARA SANGYO KAISHA, LTD., Osaka-shi Osaka 550-0002 (JP)
(72) Inventor: KIMURA, Hirohiko, Kusatsu-shi Shiga 525-0025 (JP); UEKI, Toshihiko, Kusatsu-shi Shiga 525-0025 (JP); AZUMA, Kumiko, Kusatsu-shi Shiga 525-0025 (JP); YOSHIDA, Kotaro, Kusatsu-shi Shiga 525-0025 (JP)
(74) Representative: Hartz, Nikolai
(86) International application number: PCT/JP2009/053978
(87) International publication number: WO 2009/110475

(57) **Abstract**

To provide a process for efficiently producing 3-amino-2-chloro-6-trifluoromethylpyridine.

To provide a process for producing 3-amino-2-chloro-6-trifluoromethylpyridine, comprising reacting 2-chloro-6-trifluoromethylnicotinamide with a hypochlorite in the presence of a solvent and a base, and a process for efficiently producing 2-chloro-6-trifluoromethylnicotinamide as the starting material from 2-chloro-6-trifluoromethylnicotinic acid or 2-hydroxy-6-trifluoromethylnicotinamide.

## Description

### TECHNICAL FIELD

The present invention relates to a process for producing 3-amino-2-chloro-6-trifluoromethylpyridine useful as an intermediate for e.g. an agrochemical or a medicine.

### BACKGROUND ART

3-Amino-2-chloro-6-trifluoromethylpyridine is a known compound as an intermediate for an agrochemical or a medicine, and as its production process, a production process by reduction of 2-chloro-3-nitro-6-trifluoromethylpyridine is disclosed in Patent Document 1.
Patent Document 1: JP-A-63-48268

### DISCLOSURE OF THE INVENTION

### OBJECT TO BE ACCOMPLISHED BY THE INVENTION

The production process of 3-amino-2-chloro-6-trifluoromethylpyridine is disclosed in Patent Document 1 but was insufficient as an industrial production process since the yield tends to be low.

It is an object of the present invention to provide a process for industrially producing 3-amino-2-chloro-6-trifluoromethylpyridine in high yield.

### MEANS TO ACCOMPLISH THE OBJECT

The present inventors have conducted extensive studies to accomplish the above object and as a result, they have found that it is possible to produce 3-amino-2-chloro-6-trifluoromethylpyridine in high yield by reacting 2-chloro-6-trifluoromethylnicotinamide with a hypochlorite in the presence of a solvent and a base, and also found a process for efficiently producing 2-chloro-6-trifluoromethylnicotinamide as the starting material.

That is, the present invention relates to a process for producing 3-amino-2-chloro-6-trifluoromethylpyridine, comprising reacting 2-chloro-6-trifluoromethylnicotinamide with a hypochlorite in the presence of a solvent and a base. Further, the present invention relates to a process for producing 2-chloro-6-trifluoromethylnicotinamide, comprising reacting 2-chloro-6-trifluoromethylnicotinic acid with thionyl chloride or oxalyl chloride to produce 2-chloro-6-trifluoromethylnicotinoyl chloride, and reacting the resulting 2-chloro-6-trifluoromethylnicotinoyl chloride with aqueous ammonia; and a process for producing 2-chloro-6-trifluoromethylnicotinamide, comprising reacting 2-hydroxy-6-trifluoromethylnicotinamide with phosphorus oxychloride in the presence of a base to produce 2-chloro-6-trifluoromethylnicotinonitrile, and reacting the resulting 2-chloro-6-trifluoromethylnicotinonitrile with concentrated sulfuric acid.

### EFFECTS OF THE INVENTION

According to the production process of the present invention, it is possible to industrially and efficiently produce 3-amino-2-chloro-6-trifluoromethylpyridine.

### BEST MODE FOR CARRYING OUT THE INVENTION

Now, the production process of the present invention will be described in detail. As represented by the reaction [A], it is possible to produce 3-amino-2-chloro-6-trifluoromethylpyridine represented by the formula (I) by reacting 2-chloro-6-trifluoromethylnicotinamide represented by the formula (II) with a hypochlorite in the presence of a solvent and a base.

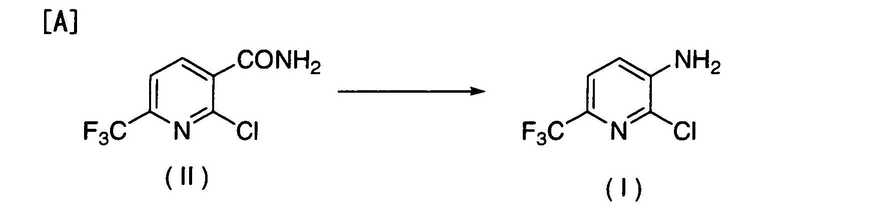

The hypochlorite to be applicable in this reaction is preferably an alkali metal hypochlorite such as sodium hypochlorite or potassium hypochlorite, and among them, sodium hypochlorite is more preferred. The amount of the hypochlorite to be used is from 0.5 to 1.5 times by mol, preferably from 0.7 to 1 time by mol, based on the compound represented by the formula (II).

The base to be applicable in this reaction may, for example, be an alkali metal hydroxide such as sodium hydroxide or potassium hydroxide; or an alkaline earth metal hydroxide such as barium hydroxide or calcium hydroxide, and the amount to be used is from 2 to 3 times by mol, based on the compound represented by the formula (II). The base is preferably sodium hydroxide.

The solvent may be any solvent so long as it is a solvent inert to the reaction, and for example, one or more types may suitably be selected from ethers such as tetrahydrofuran and dioxane; aprotic polar solvents such as acetonitrile, propionitrile, N,N-dimethylformamide, dimethylsulfoxide, hexamethylphosphoric triamide, sulfolane, dimethylacetamide and N-methyl pyrrolidone; and water. The solvent may be used in an amount of from 5 to 10 times by volume, based on the compound represented by the formula (II). The solvent is preferably water.

The reaction can be carried out at a temperature of usually from 60 to 100°C, for a reaction time of usually from about 0.5 to 1 hour.

It is possible to produce the compound represented by the formula (II) as the starting material in the above reaction [A] by the reaction [B] or [C] as shown below:

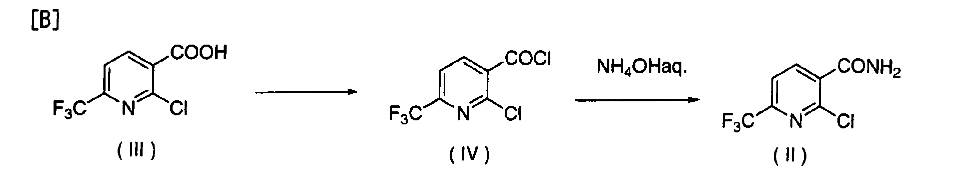

The reaction [B] is usually carried out in such a manner that 2-chloro-6-trifluoromethylnicotinic acid represented by the formula (III) is converted to 2-chloro-6-trifluoromethylnicotinoyl chloride (an acid chloride) represented by the formula (IV), and then the resulting acid chloride is treated with aqueous ammonia in the presence of a solvent to obtain the compound represented by the formula (II).

The reaction for converting to the acid chloride can usually be carried out by reacting the compound represented by the formula (III) with thionyl chloride or oxalyl chloride in an equimolar amount or more.

A solvent may be used in this reaction, and the solvent may be any solvent so long as it is a solvent inert to the reaction, and for example, one or more types may suitably be selected from halogenated hydrocarbons such as methylene chloride, chloroform, carbon tetrachloride and chlorobenzene; and aromatic hydrocarbons such as benzene, toluene and xylene.

The reaction can be carried out at a temperature of usually from 60 to 100°C, for a reaction time of usually from about 0.5 to 2 hours.

The compound represented by the formula (IV) produced in this reaction is used in the subsequent reaction with or without purification.

Then, the resulting acid chloride is usually treated with preferably from 20 to 30% aqueous ammonia in the presence of a solvent, whereby the compound represented by the formula (II) can be produced.

The aqueous ammonia may be used in an excess amount, desirably from 3 to 10 times by mol, relative to the compound represented by the formula (IV).

The solvent may be any solvent so long as it is a solvent inert to the reaction, and for example, one or more types may suitably be selected from ethers such as butyl methyl ether, tetrahydrofuran, dioxane and dimethoxyethane; and aprotic polar solvents such as acetonitrile, propionitrile, N,N-dimethylformamide, dimethylsulfoxide, hexamethylphosphoric triamide, sulfolane, dimethylacetamide and N-methyl pyrrolidone. The solvent may be used in an amount of from 1 to 3 times by volume, based on the compound represented by the formula (III).

The reaction can be carried out at a temperature of usually from 0 to 40°C, for a reaction time of usually from about 0.5 to 2 hours.

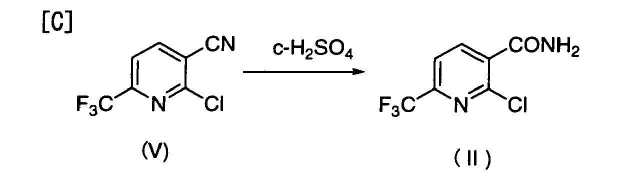

The reaction [C] is usually carried out in such a manner that 2-chloro-6-trifluoromethylnicotinonitrile represented by the formula (V) is treated with preferably from 94 to 98% concentrated sulfuric acid in an amount of from 1 to 3 times by volume.

The reaction can be carried out at a temperature of usually from 60 to 80°C, for a reaction time of usually from about 1 to 3 hours.

The compound represented by the formula (V) can be produced by the reaction [D].

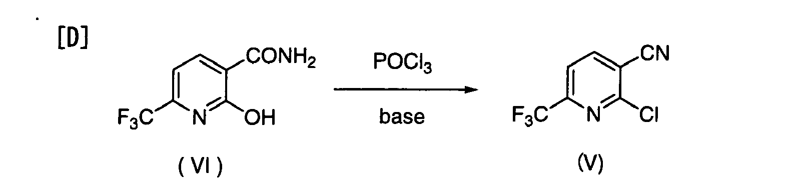

The reaction [D] is usually carried out by reacting 2-hydroxy-6-trifluoromethylnicotinamide represented by the formula (VI) with phosphorus oxychloride in the presence of a base.

The phosphorus oxychloride to be used in this reaction can be used in an amount of from 2 to 3 times by mol, based on the compound represented by the formula (VI).

As the base to be applicable in this reaction, one or more types may suitably be selected from e.g. tertiary amines such as trimethylamine, triethylamine, triisopropylamine, diisopropylethylamine, tributylamine, 4-dimethylaminopyridine, 4-pyrrolidinopyridine, N-methylmorpholine, N,N-dimethylaniline, N,N-diethylaniline, N-ethyl-N-methylaniline, 1,8-diazabicyclo[5.4.0]-7-undecene and 1,4-diazabicyclo[2.2.2]octane; quinoline; pyridine; and 2,6-dimethylpyridine. The base may be used in an amount of from 1 to 3 times by mol, based on the compound represented by the formula (V). The base is preferably tertiary amines or quinoline.

This reaction can be carried out at a temperature of usually from 100 to 160°C, preferably from 130 to 140°C, for a reaction time of usually from about 3 to 36 hours.

This reaction can be carried out in the presence of a solvent as the case requires. The solvent may be any solvent so long as it is a solvent which does not adversely affect the reaction, and for example, aromatic hydrocarbons such as toluene, xylene, chlorobenzene, 1,2-dichlorobenzene, 1,3-dichlorobenzene and 1,4-dichlorobenzene may be mentioned, and among them, 1,2-dichlorobenzene is desirable.

Further, the present invention includes the following processes:
(1) A process for producing 3-amino-2-chloro-6-trifluoromethylpyridine represented by the formula (I) by producing the compound represented by the formula (II) by the above reaction [B] and reacting the compound represented by the formula (II) with a hypochlorite in the presence of a base.
(2) A process for producing 3-amino-2-chloro-6-trifluoromethylpyridine represented by the formula (I) by producing the compound represented by the formula (II) by the above reaction [C] and reacting the compound represented by the formula (II) with a hypochlorite in the presence of a base.
(3) A process for producing 3-amino-2-chloro-6-trifluoromethylpyridine represented by the formula (I) by producing the compound represented by the formula (II) by the above reactions [D] and [C] and reacting the compound represented by the formula (II) with a hypochlorite in the presence of a base.

### EXAMPLES

The present invention will be described in further detail with reference to Examples, but it should be understood that the present invention is by no means restricted thereto.

### EXAMPLE 1 Preparation (1) of 2-chloro-6-trifluoromethylnicotinonitrile

To 300 g of 2-hydroxy-6-trifluoromethylnicotinamide, 300 mL of triethylamine was added, and 300 mL of phosphorus oxychloride was gradually dropwise added. After reaction at 130°C for 4 hours, a reaction solution cooled was gradually added to 1 L of water. Under cooling with ice, stirring was carried out for 30 minutes, and then a solid precipitated was collected by filtration and washed with 300 mL of water. The collected solid was purified by distillation under reduced pressure (120°C/18 mmHg) to obtain 247 g of 2-chloro-6-trifluoromethylnicotinonitrile (melting point: 35 to 36°C). ¹H-NMR(CDCl₃) δ=7.75(1H, d, J= 7.8 Hz), 8.21 (1H, d, J= 7.8 Hz).

### EXAMPLE 2 Preparation (2) of 2-chloro-6-trifluoromethylnicotinonitrile

To 100 g of 2-hydroxy-6-trifluoromethylnicotinamide, 86 mL of quinoline and 136 mL of phosphorus oxychloride were added in order, followed by stirring at 140°C for 24 hours. A residue obtained was cooled to room temperature and slowly added to 500 mL of water, then stirred under cooling with ice, and then a solid precipitated was collected by filtration and purified by distillation under reduced pressure (90°C/4 mmHg) to obtain 72 g of 2-chloro-6-trifluoromethylnicotinonitrile.

### EXAMPLE 3 Preparation (3) of 2-chloro-6-trifluoromethylnicotinonitrile

To a 600 ml 1,2-dichlorobenzene suspension of 300 g of 2-hydroxy-6-trifluoromethylnicotinamide, 300 mL of triethylamine was added, and 300 mL of phosphorus oxychloride was gradually dropwise added. After reaction at 140°C for 4 hours, a reaction solution cooled was gradually added to 1 L of water. An organic layer was separated and purified by distillation under reduced pressure (110 to 120°C/15 mmHg) to obtain 220 g of 2-chloro-6-trifluoromethylnicotinonitrile.

### EXAMPLE 4 Preparation (1) of 2-chloro-6-trifluoromethylnicotinamide

To 49 g of 2-chloro-6-trifluoromethylnicotinic acid, 49 mL of oxalyl chloride and a drop of DMF (dimethylformamide) were dropwise added, followed by reflux for 1 hour. After completion of reaction, an excess of oxalyl chloride was distilled off under reduced pressure to obtain crude 2-chloro-6-trifluoromethylnicotinoyl chloride (oily substance). ¹H-NMR(CDCl₃) δ=7.78(1H, d, J= 7.6 Hz), 8.50(1H, d, J= 7.6 Hz).

Then, to the obtained 2-chloro-6-trifluoromethylnicotinoyl chloride, 50 mL of THF (tetrahydrofuran) was added, and the resulting solution was gradually dropwise added to 250 mL of 28 wt% aqueous ammonia under cooling with ice. After stirring for 30 minutes, water was added thereto, followed by collection by filtration, washing with water and drying to obtain 40.7 g of 2-chloro-6-trifluoromethylnicotinamide (melting point: 215 to 217°C). ¹H-NMR(DMSO-d₆) δ= 7.97(1H, bs), 8.00(1H, d, J= 7.4 Hz), 8.18(1H, bs), 8.18(1H, d, J= 7.4 Hz).

### EXAMPLE 5 Preparation (2) of 2-chloro-6-trifluoromethylnicotinamide

To 100 g of 2-chloro-6-trifluoromethylnicotinonitrile, 140 mL of 96 vol% sulfuric acid was added, followed by stirring at 75°C for 2 hours. A reaction solution was poured in 500 mL of cooled water, and precipitated crystals were collected by filtration, washed with water and dried to obtain 108 g of 2-chloro-6-trifluoromethylnicotinamide.

### EXAMPLE 6 Preparation of 3-amino-2-chloro-6-trifluoromethylpyridine

To a mixture of 200 g of 2-chloro-6-trifluoromethylnicotinamide and 1,000 ml of water, 432 ml of a 11.6 wt% aqueous sodium hypochlorite solution and 190 ml of a 10 N aqueous sodium hydroxide solution were dropwise added in order under cooling with ice, followed by stirring at 90°C for 30 minutes. The reaction mixture was cooled to 10°C, crystals precipitated were collected by filtration, washed three times with 100 ml of cooled water, and then dried at room temperature for 24 hours to obtain 144 g of 3-amino-2-chloro-6-trifluoromethylpyridine (melting point: 96 to 97°C). ¹H-NMR(CDCl₃) δ= 4.47(2H, bs, NH₂), 7.09(1H, d, J= 7.8 Hz), 7.42(1H, d, J= 7.8 Hz).

### INDUSTRIAL APPLICABILITY

3-Amino-2-chloro-6-trifluoromethylpyridine or the like produced by the present invention is widely useful as an intermediate for an agrochemical or a medicine.

The entire disclosure of Japanese Patent Application No. 2008-053247 filed on March 4, 2008 including specification, claims and abstract is incorporated herein by reference in its entirety.

## Claims

1. A process for producing 3-amino-2-chloro-6-trifluoromethylpyridine, comprising reacting 2-chloro-6-trifluoromethylnicotinamide with a hypochlorite in the presence of a solvent and a base.

2. The process according to Claim 1, comprising reacting 2-chloro-6-trifluoromethylnicotinic acid with thionyl chloride or oxalyl chloride to produce 2-chloro-6-trifluoromethylnicotinoyl chloride, reacting the resulting 2-chloro-6-trifluoromethylnicotinoyl chloride with aqueous ammonia to produce 2-chloro-6-trifluoromethylnicotinamide, and then reacting the 2-chloro-6-trifluoromethylnicotinamide with a hypochlorite.

3. The process according to Claim 1, comprising reacting 2-hydroxy-6-trifluoromethylnicotinamide with phosphorus oxychloride in the presence of a base to produce 2-chloro-6-trifluoromethylnicotinonitrile, reacting the resulting 2-chloro-6-trifluoromethylnicotinonitrile with concentrated sulfuric acid to produce 2-chloro-6-trifluoromethylnicotinamide, and then reacting the 2-chloro-6-trifluoromethylnicotinamide with a hypochlorite.

4. A process for producing 2-chloro-6-trifluoromethylnicotinamide, comprising reacting 2-chloro-6-trifluoromethylnicotinic acid with thionyl chloride or oxalyl chloride to produce 2-chloro-6-trifluoromethylnicotinoyl chloride, and reacting the resulting 2-chloro-6-trifluoromethylnicotinoyl chloride with aqueous ammonia.

5. A process for producing 2-chloro-6-trifluoromethylnicotinamide, comprising reacting 2-hydroxy-6-trifluoromethylnicotinamide with phosphorus oxychloride in the presence of a base to produce 2-chloro-6-trifluoromethylnicotinonitrile, and reacting the resulting 2-chloro-6-trifluoromethylnicotinonitrile with concentrated sulfuric acid.
